# EUROPEAN PATENT APPLICATION

(11) **EP 1 338 292 A1**
(43) Date of publication of application: **27.08.2003**
(21) Application number: 03250991.1
(22) Date of filing: 18.02.2003
(51) Int. Cl.: A61L 27/30, A61L 27/50, A61L 27/06, A61L 27/02

(54) **Osteoconductive biomaterial and method for its production**

(30) Priority: 20.02.2002 JP 2002043632
(71) Applicant: NGK Spark Plug Co. Ltd., City of Nagoya Aichi Prefecture (JP)
(72) Inventor: Kasagua, Toshihiro, Nishikasugai-gun, Aichi (JP); Niinomi, Mitsuo, Toyohashi, Aichi (JP); Okada, Kohji, Nishikasugai-gun, Aichi (JP)
(74) Representative: West, Alan Harry

(57) **Abstract**

An osteoconductive biomaterial includes a metallic substrate and a metal oxide layer on a surface of the metallic substrate and contains, at least on the surface of the metal oxide layer, at least one chemical species selected from -PO₄H₂, -TiOH, -ZrOH, -NbOH, -TaOH and -SiOH. The metallic substrate preferably contains titanium. The metal oxide layer may be formed by heating the substrate. At least on a surface of the metal oxide layer, a specific chemical species can be formed by soaking the substrate in a phosphate buffer or a similar solution and subjecting the substrate to hydrothermal treatment at 100°C or higher and 0.1 MPa or higher. The thickness of the metal oxide layer can be, for example, about 5 µm.

## Description

The present invention relates to an osteoconductive biomaterial and a method for producing such an osteoconductive biomaterial. More particularly, the invention relates to an osteoconductive biomaterial comprising a metallic or ceramic substrate which is particularly useful for providing a biomaterial and which has an oxide layer on the free surface side of the substrate, at least a surface of the oxide layer being modified with a specific chemical species, and to a method for producing the material. The biomaterial of the present invention can serve as an artificial bone or tooth root, for example.

Metals and ceramics which have high biocompatibility have been employed to produce, for example, artificial bones and artificial tooth roots. Among these materials elemental titanium, titanium alloys, elemental zirconium, zirconium alloys, alumina, zirconia and similar materials are known to generate a remarkably thin fibrous tissue layer when implanted in living tissue. The thickness of the fibrous tissue layer increases with the degree of foreign substance level recognized by the body.

Some calcium phosphate ceramic biomaterials are known to become spontaneously chemically bonded to living bones, i.e. they are known to exhibit bioactivity when implanted in living tissue since bones and teeth are formed predominantly from calcium phosphate. Examples of such ceramic biomaterials include hydroxyapatite [Ca₁₀(PO₄)₆(OH)₂], (β-tricalcium phosphate [β-Ca₃(PO₄)₂], and β-calcium pyrophosphate (β-Ca₂P₂O₇). In addition, some glass materials are known to exhibit bioactivity. Since glass-based materials can have a wide range of compositions, the rate of bonding to living tissue, for example, bones and the chemical durability and other properties in the body can be readily controlled, which is advantageous.

When a biomaterial formed from a calcium phosphate ceramic or a glass is implanted in the body, there is formed on a surface of the biomaterial crystalline apatite that resembles living bone in terms of composition and crystallinity. Since the crystalline apatite so formed is not recognized by the body as a foreign substance, living cells populate the apatite structure, thereby initiating bone morphogenesis. This phenomenon is termed "osteoconduction." Alternatively, the crystalline apatite may be formed by soaking the biomaterial in a buffer (simulated body fluid) having inorganic ion concentrations similar to those of body fluid. The existence of osteoconductively of the relevant biomaterials can be readily determined without animal testing.

However, biomaterials other than those formed from metallic materials or calcium phosphate ceramic materials are known to form no direct chemical bond to bone tissue when implanted into the body. Accordingly, when such biomaterials are employed as artificial bones or artificial tooth roots, which require strong bonding to living bones, the bonding strength must be enhanced. To this end, it has been proposed to use a biomaterial in which a ceramic coating film comprising calcium phosphate (for example, hydroxyapatite or β-tricalcium phosphate) is formed on a surface of a metallic substrate or ceramic substrate (other than calcium phosphate).

Generally, calcium phosphate ceramic coating films are formed on substrates such as metallic substrates by plasma-spraying. However, substantially no chemical bonds are then formed between the substrate and the coating. Consequently, when the resulting biomaterial is implanted in living tissue, the coating film often peels off the substrate at an early stage.

Glass materials raise the problem that the safety of SiO₂―their predominant component―in the living body has not yet been confirmed. Literature, such as Japanese Patent Application Laid-Open (kokai) No. 2001-80936, discloses a biomaterial comprising a titanium-based metallic substrate strongly coated with calcium phosphate-containing glass for solving that problem. However, the resulting coating film is readily peeled off the substrate when the biomaterial is implanted in living tissue, which is problematic.

The present invention has as its overall object to solve the above problems in conventional techniques. Thus, a specific object of the present invention is to provide an osteoconductive biomaterial which exhibits sufficient osteoconductivity and which readily forms a chemical bond to living tissue (e.g., living bone) when implanted in the body. Another object of the invention is to provide a method for producing such an osteoconductive biomaterial.

In accordance with the invention, there is provided an osteoconductive biomaterial comprising a substrate having a surface, said surface comprising oxide material, and which contains at least on a surface of said oxide material a layer containing at least one species selected from the group consisting of -PO₄H₂, -TiOH, -ZrOH, -NbOH, -TaOH and -SiOH.

In accordance with a first specific aspect of the present invention, the osteoconductive biomaterial comprises a metallic substrate having a metal oxide layer on a surface thereof and contains, at least on a surface of the metal oxide layer, a layer containing at least one chemical species selected from PO₄H₂, TiOH, ZrOH, NbOH, TaOH and SiOH. In this embodiment of the invention, the metallic substrate may advantageously contain titanium.

In accordance with a second specific aspect of the invention, the osteoconductive biomaterial comprises an oxide ceramic substrate which contains at least on a surface of the oxide ceramic a layer containing at least one chemical species selected from -PO₄H₂, -TiOH, -ZrOH, -NbOH, -TaOH and -SiOH. In this embodiment of the invention, the oxide ceramic substrate may contain at least one oxide selected from titanium oxide, zirconium oxide, niobium oxide, tantalum oxide, silicon oxide, and phosphorus oxide. In this embodiment also the oxide may contain at least one metallic element selected from sodium, magnesium, aluminum, potassium, calcium, iron, zinc, yttrium, tin, lanthanum, silver, gold and platinum.

In accordance with a third specific aspect of the present invention, the osteoconductive biomaterial comprises a non-oxide ceramic substrate having an oxide ceramic layer on a surface thereof and which contains, at least on a surface of the oxide ceramic layer, a layer containing at least one chemical species selected from -PO₄H₂, -TiOH, -ZrOH, -NbOH, -TaOH and -SiOH. In this third embodiment of the invention, the oxide ceramic layer may contain at least one oxide selected from titanium oxide, zirconium oxide, niobium oxide, tantalum oxide, silicon oxide, and phosphorus oxide. In this third embodiment, the oxide may contain at least one metallic element selected from sodium, magnesium, aluminum, potassium, calcium, iron, zinc, yttrium, tin, lanthanum, silver, gold and platinum.

In these three specific aspects of the present invention, it is not necessary for the specified chemical species to cover completely the surface of the oxide material. It is sufficient that only a part of the surface of the oxide material carries the layer which contains at least one of the specified chemical species in order to provide osteoconductivity.

The osteoconductive biomaterial of the present invention may be advantageously employed as a material for providing artificial bones and artificial tooth roots.

In accordance with a fourth aspect of the present invention, there is provided a method for producing an osteoconductive biomaterial according to the first embodiment described above, the method comprising soaking the metallic substrate on which the metal oxide layer is formed in a liquid selected from water, aqueous solutions containing hydroxide, and aqueous solutions containing at least one of phosphoric acid and phosphate salts; and hydrothermally treating the soaked substrate at a temperature of 100°C or higher under a pressure of 0.1 MPa or higher.

In accordance with a fifth aspect of the present invention, there is provided a method for producing an osteoconductive biomaterial according to the second embodiment described above, the method comprising soaking the oxide ceramic substrate in a liquid selected from water, aqueous solutions containing hydroxide, and aqueous solutions containing at least one of phosphoric acid and phosphate salts; and hydrothermally treating the soaked substrate at a temperature of 100°C or higher under a pressure of 0.1 MPa or higher.

In accordance with a sixth aspect of the present invention, there is provided a method for producing an osteoconductive biomaterial according to the third amendment described above, the method comprising soaking the non-oxide ceramic substrate on which the oxide ceramic layer is formed in a liquid selected from water, aqueous solutions containing hydroxide, and aqueous solutions containing at least one of phosphoric acid and phosphate salts; and hydrothermally treating the soaked substrate at a temperature of 100°C or higher under a pressure of 0.1 MPa or higher.

The invention is described below in greater detail by way of example only with reference to the accompanying drawings, in which
Fig. 1 is a cross-sectional schematic view showing an osteoconductive biomaterial including a metallic substrate;
Fig. 2 is a cross-sectional schematic view showing an osteoconductive biomaterial including an oxide ceramic substrate; and
Fig. 3 is a cross-sectional schematic view showing an osteoconductive biomaterial including a non-oxide ceramic substrate.

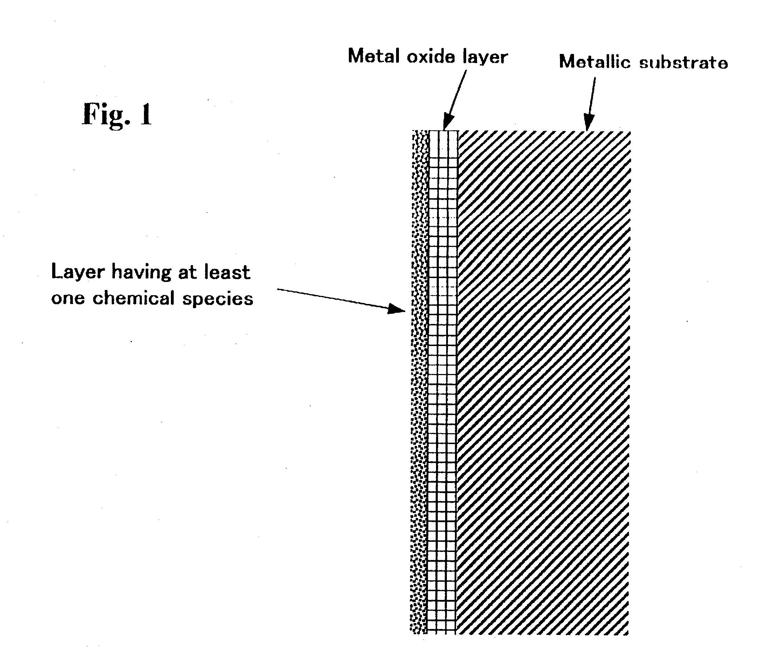

An osteoconductive biomaterial containing a metallic substrate in accordance with the first embodiment of the present invention is shown schematically in Fig. 1 of the accompanying drawings.

Although no particular limitation is imposed on the metal species of the metallic substrate, the substrate preferably contains titanium, which has excellent biocompatibility, in consideration that the metallic substrate is intended for use in a biomaterial. The metal oxide layer is readily formed on a surface of the metallic substrate by means of heating the metallic substrate or by means of other treatment. The titanium-containing substrate can be formed from elemental titanium or any of various titanium alloys.

Examples of preferred titanium alloys include T1-6Al-4V and Ti-29Nb-13Ta-4.6Zr. Among them, titanium alloys containing β-titanium are preferred, since these alloys have an elastic modulus similar to that of living bone and high mechanical strength. The metallic substrate may also be formed from titanium and a readily oxidizable metal. Alternatively, the metallic substrate may be formed from a readily oxidizable metal other than titanium (i.e., containing no titanium). Examples of readily oxidizable metals include zirconium, niobium, tantalum, molybdenum and tin. Thus, in addition to a titanium-containing metallic substrate, there may be employed a zirconium-containing, niobium-containing, tantalum-containing, molybdenum-containing and tin-containing metallic substrates, and similar substrates.

No particular limitation is imposed on the thickness of the metal oxide layer on the surface of the metal substrate. However, the thickness is preferably 100 µm or less, more preferably 20 µm or less and most preferably 3 to 10 µm. Thicknesses greater than 100 µm are not preferred, since the metal oxide layer may then sometimes peel off the metallic substrate.

No particular limitation is imposed on the method for forming the metal oxide layer, and the layer may be formed by heating the metallic substrate. Specifically, the metallic substrate may be heated in an oxygen-containing atmosphere, to thereby form the metal oxide layer. Heating may be carried out using any of a variety of heating furnaces, such as a muffle furnace, a controlled-atmosphere firing furnace or an infrared beam heating furnace. No particular limitation is imposed on the temperature and time of heating so long as a metal oxide layer of predetermined thickness can be formed. However, when a metallic substrate is heated at 1,000°C or higher, the metal oxide layer so formed may attain a thickness greater than 100 µm, possibly causing the metal oxide layer to peel off the metallic substrate. Therefore, heating is preferably carried out at lower than 1,000°C. Alternatively, a metal oxide layer can be formed through anodization of a metallic substrate; i.e., electrolysis at a predetermined potential by use of the metallic substrate as an anode.

The metal oxide layer contains, at least on a surface thereof, hydroxy groups which impart hydrophilicity to the surface. Hydrophilicity is an important factor for a biomaterial having biocompatibility. In addition, the surface contains a specific chemical species which is formed by bonding a hydroxy group to a certain element, the chemical species serving as a functional group for providing nuclei of bone-like apatite crystals. Examples of the chemical species include -PO₄H₂, -TiOH, -ZrOH, -NbOH, -TaOH and -SiOH. To each chemical species there are coordinated calcium ions along with phosphate ions contained in body fluid or simulated body fluid thereby to form nuclei for bone-like apatite crystals. Crystal growth proceeds from the nuclei while ionic species contained in the body fluid or simulated body fluid are consumed. As a result, at least a surface of the metal oxide layer is covered with bone-like apatite crystals, thereby yielding a biomaterial exhibiting a satisfactory osteoconductive properties.

Among these chemical species, -PO₄H₂ and -TiOH are particularly preferred, by virtue of their high rate of formation of bone-like apatite crystals. The rate of formation of bone-like apatite crystals increases with increasing proton releasability of the chemical species. Thus, PO₄H₂ is most preferred, since -PO₄H₂ is more easily dissociated. These chemical species dissociate protons through contact with body fluid or simulated body fluid, and calcium ions and phosphate ions are successively coordinated to the thus-formed proton-dissociated sites, thereby promoting formation of nuclei for bone-like apatite crystals. Thus, chemical species which readily dissociate protons are preferred, in view of the increased rate of formation of bone-like apatite crystals.

The chemical species may be present on the surface of the metal oxide layer, or may be present within a depth of up to 1 µm from the surface, particularly a depth falling within a range of 0.1 to 0.8 µm. Generally, specific chemical species are difficult to incorporate into a depth greater than 1 µm.

The specific chemical species may be formed, at least on the surface of the metal oxide layer, by soaking the metallic substrate on which the metal oxide layer has been formed, in a liquid selected from water and aqueous solutions containing hydroxide and aqueous solutions containing at least one of phosphoric acid and phosphate salts; and heating. No particular limitation is imposed on the water to be used, and pure water, ion exchange water, etc, can be employed. When water is used, there are formed hydroxy groups which are chemically bonded to the metal oxide layer. Examples of the above hydroxide include NaOH, KOH and Ca(OH)₂. When a hydroxide-containing aqueous solution is used, hydroxy groups can be readily formed at least on a surface of the metal oxide layer. In this case, an analogous group in which the hydrogen atom of the hydroxy group is substituted by a metallic element of the corresponding hydroxide is also formed. Such a metal-substituted group must be transformed back into a hydroxy group through neutralization by addition of any amount of acid. Examples of phosphate salts include sodium monohydrogenphosphate, sodium dihydrogenphosphate and potassium hydrogenphosphate. When an aqueous solution containing phosphoric acid or a phosphate salt (the salt requires neutralization after completion of soaking), hydroxy groups and -PO₄H₂ can be formed.

The metallic substrate on which the metal oxide layer has been formed is suitably soaked at 100°C or higher, preferably 100 to 200°C. Since soaking is carried out in a sealed container such as a pressure container, the pressure during soaking suitably reaches 0.1 MPa or higher, depending on the soaking temperature. By hydrothermal treatment, hydroxy groups are formed at least on a surface of the metal oxide layer. When an aqueous solution containing phosphoric acid and/or a phosphate salt is used, PO₄H₂ groups are formed through substitution of protons by phosphate ions and bonded to the metal oxide layer. The water or the aqueous solution may contain a variety of ionic species such as Ca²⁺, Mg²⁺, Na⁺, K⁺, HCO₃⁻, HPO₄⁻, Cl⁻ and SO₄²⁻, which are generally contained in simulated body fluid.

Formation of chemical species such as -PO₄H₂, -TiOH, -ZrOH, -NbOH, -TaOH and -SiOH at least on the surface of the metal oxide layer can be confirmed through X-ray photoelectron spectroscopy. The presence of hydroxy groups as well as the amount of hydroxy groups can be confirmed and determined on the basis of binding energy gap in terms of the inner shell electrons of the oxygen atom, which is determined through, for example, the corresponding O₁ₛ spectrum. Through etching of the surface using argon ions and subsequent measurement, formation of hydroxy groups can be confirmed only in the vicinity of the surface. In the case where -PO₄H₂ groups are bonded, when O-related and P-related spectra are measured after etching of the surface by using argon ions, formation of -PO₄H₂ groups can be confirmed only in the vicinity of the surface.

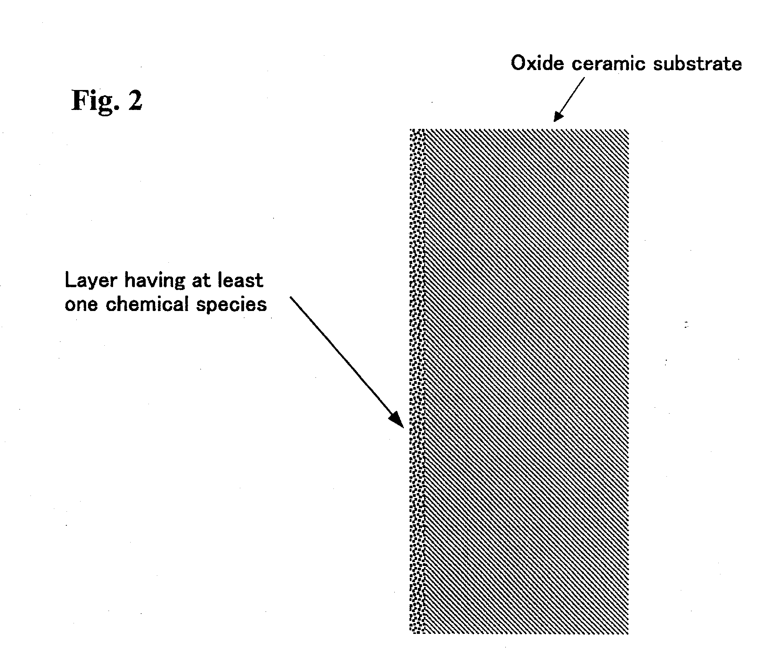

An osteoconductive biomaterial containing an oxide ceramic substrate according to the second embodiment of the present invention is shown schematically in Fig. 2 of the accompanying drawings.

The oxide ceramic substrate preferably contains at least one oxide selected from titanium oxide, zirconium oxide, niobium oxide, tantalum oxide, silicon oxide and phosphorus oxide. Of these, titanium oxide, zirconium oxide and phosphorus oxide are more preferred. Zirconium oxide is particularly preferred for providing hard tissue substitutes such as artificial bone or artificial tooth roots, since zirconium oxide has remarkably high mechanical strength and toughness.

The oxide preferably contains at least one metallic element selected from sodium, magnesium, aluminum, potassium, calcium, iron, zinc, yttrium, tin, lanthanum, silver, gold and platinum. When the substrate is formed from an oxide containing any of these metallic elements, hydroxy groups are readily bonded to a surface of the substrate.

The oxide ceramic substrate may be replaced by a metallic substrate coated with an oxide ceramic for enhancing mechanical strength. In this case, the oxide ceramic coating may comprise a vitreous material, or both a vitreous material and a crystalline material. The above composite substrate may comprise for example, a titanium-containing metallic substrate coated with a vitreous or vitreous-crystalline coating containing an oxide such as CaO, P₂O₅, Na₂O or TiO₂.

In a similar manner to the embodiment involving the metallic substrate, the oxide ceramic substrate contains, at least on the surface thereof, hydroxy groups and a specific chemical species, thereby yielding a biomaterial exhibiting osteoconductive properties. As the chemical species, -PO₄H₂ and TiOH are preferred by virtue of their high rate of formation of bone-like apatite crystals, with -PO₄H₂ being particularly preferred, for the reasons described above. Introduction of such chemical species promotes formation of nuclei for bone-like apatite crystals, thereby further increasing the rate of formation of bone-like apatite crystals.

Again for the reasons stated above in relation to the embodiment involving the metallic substrate, the depth to which the chemical species is to be incorporated is limited since the chemical species is difficult to incorporate to a depth greater than that specified above.

Hydroxy groups and the specific chemical species can be formed on the surface of an oxide ceramic substrate through hydrothermal treatment performed in a manner similar to that described above in connection with the embodiment utilizing the metallic substrate. Hydrothermal treatment conditions including apparatus, water or aqueous solution, temperature and pressure are similar to those described above. When an aqueous solution containing for example NaOH, KOH or Ca(OH)₂ is used, hydroxy groups are formed at high efficiency. In this case, the resulting analogous group in which the hydrogen atom of the hydroxy group is substituted by a metallic element of the corresponding hydroxide must be transformed back into a hydroxy group through neutralization by addition of any amount of acid. When an aqueous solution containing phosphoric acid and/or a phosphate salt is used, -PO₄H₂ can also be formed. Water or the aqueous solutions may contain a variety of ionic species such as Ca²⁺, Mg²⁺, Na⁺, K⁺, HCO₃⁻, HPO₄⁻, Cl⁻ and SO₄²⁻, which are contained in, for example, simulated body fluid.

Again. the existence of the specific chemical species can be confirmed through X-ray photoelectron spectroscopic analysis.

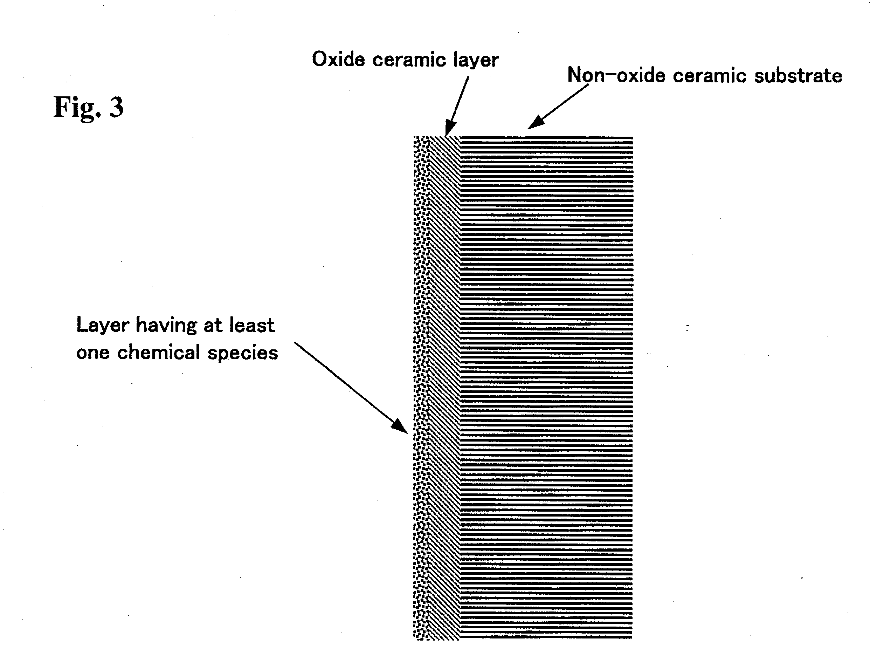

An osteoconductive biomaterial containing a non-oxide ceramic substrate according to the third embodiment of the present invention is shown schematically in Fig. 3 of the accompanying drawings.

Examples of non-oxide ceramics from which the substrate may be formed include nitride ceramics such as silicon nitride and carbide ceramics such as silicon carbide. Such a ceramic substrate must be coated with the oxide ceramic layer. The oxide ceramic layer can be formed on the surface of the substrate through sputtering by use of a target formed of an oxide such as titanium oxide, zirconium oxide, niobium oxide, tantalum oxide, silicon oxide and phosphorus oxide. Alternatively, the oxide ceramic layer can be formed by a wet method such as the sol-gel method.

No particular limitation is imposed on the thickness of the metal oxide layer on the surface of the non-oxide ceramic substrate. However, the thickness is preferably 100 µm or less, more preferably 20 µm or less and most preferably 3 to 10 µm. Thicknesses greater than 100 µm are not preferred, since the oxide ceramic layer may then peel off the non-oxide ceramic substrate.

In a similar manner to the embodiment involving the metallic substrate, the oxide ceramic layer contains, at least on the surface thereof, hydroxy groups and a specific chemical species, thereby yielding a biomaterial exhibiting excellent osteoconductive properties. As the chemical species, -PO₄H₂ and -TiOH are preferred, by virtue of their high rates of formation of bone-like apatite crystals, with -PO₄H₂ being particularly preferred, for the reasons described above. Introduction of such chemical species promotes formation of nuclei for bone-like apatite crystals, thereby further increasing the rate of formation of bone-like apatite crystals.

Again, for the reasons stated above in relation to the embodiment involving the metallic substrate, the depth to which the chemical species is to be incorporated is limited since the chemical species is difficult to incorporate to a depth greater than that specified above.

Hydroxy groups and the specific chemical species can be formed on the surface of the oxide ceramic layer formed on the non-oxide ceramic substrate through hydrothermal treatment performed in a manner similar to that described above in connection with the embodiment utilizing the metallic substrate. Hydrothermal treatment conditions including apparatus, water or aqueous solution, temperature, and pressure are similar to those described above. When an aqueous solution containing NaOH, KOH or Ca(OH)₂, is used, hydroxy groups can be formed at high efficiency. In this case, an analogous group in which the hydrogen atom is substituted by a metallic element must be transformed back into a hydroxy group through neutralization. When an aqueous solution containing phosphoric acid and/or a phosphate salt is used, -PO₄H₂ groups can also be formed. Water or the aqueous solution may contain a variety of ionic species such as Ca²⁺, Mg²⁺, Na⁺, K⁺, HCO₃⁻, HPO₄⁻, Cl⁻ and SO₄²⁻, which are contained in, for example, simulated body fluid.

Again, the existence of the specific chemical species can be confirmed through X-ray photoelectron spectroscopic analysis.

The osteoconductive biomaterials according to the present invention are preferably employed as materials which are used in contact with the living body, particularly as artificial bones and artificial tooth roots, which repeatedly and frequently receives a heavy load within the body. The use of such biomaterials in the body can secure a long-term, stable bonding state to the body, along with safety.

X-ray photoelectron spectroscopic analysis has revealed that specific chemical species contained at least on the surface of each biomaterial produced according to the present invention imparts osteoconductive properties to the biomaterial exclusively within a depth of up to 1 µm from the surface. The surface portion containing the chemical species is confirmed to be non-peeled during customary peeling tests such as tape tests and scratch tests.

The following Examples illustrate the invention.

### Example 1

A titanium-containing metallic substrate (Ti-29Nb-13Ta-4.6Zr alloy plate) was washed with acetone and then dried. The dried substrate was placed in a muffle furnace and heated to 800°C in air at 5°C/minute. The substrate was maintained at 800°C for one hour for heat treatment. The furnace was then switched off and the substrate was allowed to cool in the furnace. Electron beam microprobe microanalysis revealed that a metal oxide layer was formed from the surface of the heat-treated substrate to a depth of about 5 µm. X-ray photoelectron spectroscopic analysis revealed that the oxide comprised titanium oxide, niobium oxide, tantalum oxide and zirconium oxide.

Sodium monohydrogenphosphate (2.84 g) and sodium dihydrogenphosphate (0.6 g) were dissolved in water (125 mL) to thereby prepare a phosphate buffer, and the buffer was placed in a silica glass container. The substrate provided with the metal oxide layer was soaked in the aqueous solution. Subsequently, the glass container was placed in a pressure container of stainless steel, and the container was sealed. The pressure container was heated by means of an electric heater wound around the container such that the phosphate buffer was heated to 120°C. Through heating, the internal pressure of the container reached 0.2 MPa. After maintaining the conditions for one hour, electric heating was stopped, and the substrate in the buffer was allowed to cool. Subsequently, the substrate was removed from the aqueous solution and placed in a thermostat (controlled to 60°C) container for drying. X-ray photoelectron spectroscopic analysis revealed that P formed chemical bonds to a surface portion (from the surface to a depth of about 0.5 µm) of the metal oxide layer after hydrothermal treatment, and that a large number of hydroxy groups are contained in the surface portion. The observation indicates that the surface portion of the metal oxide layer contains -TiOH, -NbOH, -TaOH, -ZrOH and -PO₄H₂.

The substrate which had been subjected to hydrothermal treatment was soaked in a simulated body fluid containing the following ions: Cₐ²⁺, Mg²⁺, Na⁺, K⁺, HCO₃⁻, HPO₄⁻, Cl⁻ and SO₄²⁻, and the fluid was placed in a 37°C thermostat for 10 days. Thereafter, the substrate was removed from the simulated body fluid and gently washed with distilled water. The substrate was dried, the surface of the substrate was observed under a scanning electron microscope, and a large number of petal-like crystals were identified. The crystals were found to predominantly contain calcium and phosphorus with a small amount of magnesium and to have an atomic ratio of Ca/P of 1.52. The crystals were identified as bone-like apatite crystals, on the basis of the similarity of the atomic ratio and crystal morphology characteristic to those of known bone-like apatite crystals. Accordingly, the biomaterial is considered to have function for forming bone-like apatite crystals in simulated body fluid; i.e., to have osteoconductive properties.

### Comparative Example 1

A titanium-containing metallic substrate which had not been provided with a metal oxide layer was subjected to hydrothermal treatment similar to that described in Example 1 above and the treated substrate was soaked in a simulated body fluid in a similar manner. Observation of the surface of the substrate revealed no new products such as crystals.

### Comparative Example 2

A titanium-containing metallic substrate was provided with a metal oxide layer in a manner similar to that described in Example 1 above and the resultant substrate was soaked in a simulated body fluid in a manner similar to that of Example 1 without previous hydrothermal treatment. Observation of a surface of the substrate revealed no new products such as crystals.

### Example 2

An oxide ceramic substrate (3 mol% yttrium oxide-containing zirconia ceramic plate) was washed with acetone and then dried. Subsequently, a phosphate buffer as described in Example 1 was placed in a silica glass container and the oxide ceramic substrate was soaked in the aqueous buffer solution. Subsequently, the glass container was placed in a pressure container of stainless steel, the container was sealed and was heated by means of an electric heater wound around the container such that the phosphate buffer was heated to 180°C. Through heating, the internal pressure of the container reached 1 MPa. After maintenance of the conditions for one hour, electric heating was stopped, and the substrate in the buffer was allowed to cool. Subsequently, the substrate was removed from the aqueous solution and placed in a thermostat (controlled to 60°C) container for drying. X-ray photoelectron spectroscopic analysis revealed that P formed chemical bonds to the surface portion (from the surface to a depth of about 0.2 µm) of the oxide ceramic substrate after hydrothermal treatment, and that a large number of hydroxy groups are contained in the surface portion. The observation indicates that the surface portion of the oxide ceramic substrate contains -ZrOH and -PO₄H₂.

In a manner similar to that described in Example 1, the substrate was soaked in a simulated body fluid, and washed and dried. Observation of a surface of the substrate under a scanning electron microscope enabled a large number of petal-like crystals to be identified. The crystals were found to predominantly contain calcium and phosphorus with a small amount of magnesium and to have an atomic ratio of Ca/P of 1.53. The crystals were identified as bone-like apatite crystals, on the basis of the similarity of the atomic ratio and crystal morphology characteristic to those of known bone-like apatite crystals. Accordingly, the biomaterial is considered to have function for forming bone-like apatite crystals in simulated body fluid; i.e., to have osteoconductive properties.

The osteoconductive biomaterials according to the present invention exhibit osteoconductive properties when implanted in living tissue. When the metallic substrate contains titanium, biocompatibility is further enhanced. When the oxide ceramic substrate contains an oxide including a specific element and the oxide contains a specific metallic element, osteoconductive properties can be further enhanced. Also, when the oxide ceramic layer contains an oxide including a specific element and the oxide contains a specific metallic element, osteoconductive properties can be further enhanced.

## Claims

1. An osteoconductive biomaterial comprising a substrate having a surface, comprising oxide material and which contains at least on a surface of the oxide material a layer containing at least one species selected from -PO₄H₂, -TiOH, -ZrOH, -NbOH, -TaOH and -SiOH.

2. An osteoconductive biomaterial according to claim 1, comprising a metallic substrate and a metal oxide layer which is provided on a surface or the metallic substrate and which contains, at least on a surface of the metal oxide layer, a layer having at least one chemical species selected from -PO₄H₂, -TiOH, -ZrOH. -NbOH, -TaOH and -SiOH.

3. An osteoconductive biomaterial according to claim 2, wherein the metallic substrate contains titanium.

4. An osteoconductive biomaterial according to claim 1, comprising an oxide ceramic substrate which contains, at least on a surface thereof, a layer having at least one chemical species selected from -PO₄H₂, -TiOH, -ZrOH, -NbOH, -TaOH and -SiOH.

5. An osteoconductive biomaterial according to claim 4, wherein the oxide ceramic substrate contains at least one oxide selected from titanium oxide, zirconium oxide, niobium oxide, tantalum oxide, silicon oxide and phosphorus oxide.

6. An osteoconductive biomaterial according to claim 4 or claim 5, wherein the oxide contains at least one metallic element selected from sodium, magnesium, aluminum, potassium, calcium, iron, zinc, yttrium, tin, lanthanum, silver, gold and platinum.

7. An osteoconductive biomaterial according to claim 1, comprising a non-oxide ceramic substrate and an oxide ceramic layer which is provided on a surface of the non-oxide ceramic substrate and which contains, at least on a surface of the oxide ceramic layer, a layer having at least one chemical species selected from -PO₄H₂, -TiOH, -ZrOH, -NbOH, -TaOH and -SiOH.

8. An osteoconductive biomaterial according to claim 7, wherein the oxide ceramic layer contains at least one oxide selected from titanium oxide, zirconium oxide, niobium oxide, tantalum oxide, silicon oxide and phosphorus oxide.

9. An osteoconductive biomaterial according to claim 7 or claim 8, wherein the oxide contains at least one metallic element selected from sodium, magnesium, aluminum, potassium, calcium, iron, zinc, yttrium, tin, lanthanum, silver, gold and platinum.

10. An osteoconductive biomaterial according to any one of claims 1 to 9, which is employed as a material for an artificial bone or an artificial tooth root.

11. A method for producing an osteoconductive biomaterial according to claim 2, the method comprising soaking the metallic substrate on which the metal oxide layer has been formed in a liquid selected from water, aqueous solutions containing hydroxide, and aqueous solutions containing at least one of phosphoric acid and phosphate salts; and hydrothermally treating the soaked substrate at 100°C or higher and 0.1 MPa or higher.

12. A method for producing an osteoconductive biomaterial according to claim 4, the method comprising soaking the oxide ceramic substrate in a liquid selected from water, aqueous solutions containing hydroxide, and aqueous solutions containing at least one of phosphoric acid and phosphate salts; and hydrothermally treating the soaked substrate at 100°C or higher and 0.1 MPa or higher.

13. A method for producing an osteoconductive biomaterial according to claim 7, the method comprising soaking the non-oxide ceramic substrate on which the oxide ceramic layer has been formed in a liquid selected from water, aqueous solutions containing hydroxide and aqueous solutions containing at least one of phosphoric acid and phosphate salts; and hydrothermally treating the soaked substrate at 100°C or higher and 0.1 MPa or higher.
